# EUROPEAN PATENT SPECIFICATION

(11) **EP 1 432 381 B1**
(45) Date of publication and mention of the grant of the patent: **11.10.2006**
(21) Application number: 02760035.2
(22) Date of filing: 27.09.2002
(51) Int. Cl.: A61K 6/04, C22C 5/06

(54) **DENTAL ALLOY WITH SILVER CONTENT**
DENTALLEGIERUNG MIT HOHEM SILBERGEHALT
ALLIAGE DENTAIRE PRESENTANT UNE TENEUR EN ARGENT

(30) Priority: 01.10.2001 CH 180401
(43) Date of publication of application: 30.06.2004
(60) Divisional application: 06007558.7
(73) Proprietor: CENDRES ET METAUX S.A., CH-2501 Bienne (CH); Wegold Edelmetalle AG, 90530 Wendelstein (DE)
(72) Inventor: GUO-HUANG, Kangping, CH-2074 Marin (CH); BALTZER, Niklaus, CH-2504 Biel/Bienne (CH); DÖRFLER, Beat, CH-2533 Evilard (CH)
(74) Representative: AMMANN PATENTANWÄLTE AG BERN
(86) International application number: PCT/CH2002/000539
(87) International publication number: WO 2003/028669

(56) References cited:
- EP-A2- 0 178 506
- DE-C1- 19 713 925
- US-A- 4 804 517
- US-A- 5 290 371
- US-A- 5 462 437
- PATENT ABSTRACTS OF JAPAN vol. 2000, no. 10, 17 November 2000 (2000-11-17) & JP 2000 192170 A (NIPPON SHIKEN KOGYO KK), 11 July 2000 (2000-07-11)
- DATABASE WPI Section Ch, Week 199345 Derwent Publications Ltd., London, GB; Class D21, AN 1993-357962 XP002186571 -& NL 9 200 566 A (ELEPHANT EDELMETAAL BV) , 18 October 1993 (1993-10-18)

## Description

The present invention refers to a dental alloy with silver content according to the preamble of claim 1.

A large number of such silver alloys are known, which are mainly used because they are less expensive than high gold alloys. The different silver alloys are adapted to different kinds of indications. In these alloys, an elevated palladium content is required to compensate for the high thermal expansion coefficient of silver if they are to be veneered by ceramic material.

As an alternative, alloys of high gold content have been veneered by ceramics.

Gold alloys show a desired yellow appearance, yet have a low melting point not well suited to veneering by ceramics as the material is loosing its shape while the ceramic material is fused to the alloy body. The gold or gold alloys also have a relatively high density, hence more of the rather expensive material is needed.

Regarding silver palladium alloy, a superficial oxide layer is necessary to secure the fast adherence of the ceramic material on the silver palladium alloys. This oxide layer is produced by a particular oxide firing step and shows a rather dark colour which would impair the optical appearance of the veneered area. Even if special opaque layers of ceramic are applied, the dark colour is perceivable in the boundary areas where the ceramic layer is thin, and of course at the periphery of the ceramic veneer. In order to avoid this effect, the surface may be specially treated so that the oxide gets a light or nearly white colour, yet this treatment again increases the overall costs and does avoid that the product shows an unnatural appearance resembling steel.

Other drawbacks of silver palladium alloys are that delicate structures are difficult be casted in comparison with gold, and the neutral, metallic appearance where the surface of the alloy is visible, i.e. at the non-veneered surface areas.

Regarding the incorporation of palladium, not only contravenes the use of this expensive noble metal the goal to provide a non-expensive alloy, it renders its price also prone to large changes due to the higly volatile market of the platinum metals.

Another aspect is the availability of ceramics which can be fused to an alloy body using lower firing temperatures. These ceramics, however, show also a higher thermal expansion, generally higher than 14.5 * 10⁻⁶ K⁻¹. For a stable and rigid bond among ceramic and alloy, the thermal expansion of alloy and ceramic has to be matched, the thermal expansion of the ceramic preferably being slightly less than that of the alloy, e.g. by about 1 K⁻¹ or less.

JP-A-2000 192 170 proposes an alloy based mainly on silver, palladium and gold. The proposed total contents of copper and zinc according to the embodiments is at least 11 %, and the alloys have thermal expansion coefficients of at least 18 * 10⁻⁶ K⁻¹. It is not mentioned that ceramics are capable to be fused to a body of the these alloys, but additionally to dentistry, their use is extended to repairing decorative parts, electrical contacts etc. Finally, the high thermal expansion of the exemplary alloys forbids the use for ceramic-fused-to-metal dental devices.

NL-A-9 200 566 relates to a dental alloy, on which ceramics may be fused. The lowest melting range of the embodiments given is 990-1110 °C, and the thermal expansion coefficients of the embodiments are (15.5 to 17.5) * 10⁻⁶ K⁻¹. The compositions of the embodiments of the alloy with respect to silver, palladium and indium is characterized by the values (Ag/Pd/In, weight percentages): 43/44/8.5; 68.2/22/6.5; 49.5/40/7.5. Gold is contained at most by 1 %.

EP-A-0 178 506 proposes similar silver palladium alloys showing a solidus temperature of 1075 °C or more. One characteristic feature of these alloys is the content of at most 0.5 % gold or the absence of gold.

DE-A-197 13 925 discloses silver palladium alloys with at most 1 % gold. The alloys show solidus temperatures of at least 1015 °C.

Else, the alloys according to the three aforementioned publications show the general drawbacks for silver palladium alloy as set forth above.

Alloys for general applications in dentistry and jewelry are described in US-4,804,517. In general, a yellowish, gold-like color is aimed at and a hardness required for dental restorations suitable for at least moderate stress. Particularly with respect to color, it is disclosed that an intermetallic phase corresponding about to the composition PdIn produces a yellow color, whereas a transition to PdIn₂ weakens the color again.

Therefore, it is desirable and thus also an object of the invention to provide a dental alloy with silver content that is suitable for a large number of applications ranging from inlays to long-span bridgework and offers improved mechanical properties, particularly a higher stability and corrosion resistance, and is compatible with low fusing ceramics with high thermal expansion coefficient, i.e. suitable for veneering. Furthermore, the alloy shall have a yellow colour, i. e. resemble gold.

This object is attained by the alloy according to claim 1. If the dental alloy is to meet very high requirements with respect to mechanical stability, the dental alloy according to claim 2 is preferred.

The other claims define preferred embodiments of the alloys and uses thereof and products obtained.

### Detailed Description of the Invention

The invention will be explained in more detail hereinafter with reference to exemplary embodiments. As illustrated by the silver dental alloys of the prior art, and as it is known to those skilled in the art from tests, books and the like, certain elements besides the noble metal components will provide certain effects, and it is known that e.g. zinc will harden an alloy, or iridium may be used as a grain refiner. The same applies to additional metals such as rhodium, ruthenium and the like.

Within the whole specification including the claims, percentages are by weight.

Surprisingly, extensive tests have now shown that the alloy described hereinafter, by the mere addition of palladium and indium to an alloy containing silver and gold within the indicated limits, provides a high corrosion resistance, improved mechanical properties and a coefficient of thermal expansion that makes it suitable for veneering particularly by low-fusing ceramics having a coefficient of thermal expansion ranging from 14.5 to 16.5. Furthermore, the alloy has ameliorated casting properties so that delicate surface structures can be mold without difficulty. Subsequent grinding and polishing for the finish of the cast object and elaborating minute details can be readily done without applying undue force. Still to mention that the alloy shows a yellowish colour, which is preferred for esthetical reasons. Ceramic material may be fused to the alloy directly, the problem with the dark coloured oxide surface layer does not arise.

The alloy is composed of 35.5 to 62.5 % silver, 4.1 to 11.0 % indium, 5 to 37.0 % gold, 0 to 40.0 % platinum, 0 to 35 % palladium, 0 to 9.5 % zinc, tin, gallium, copper, 0 to 2.0 % iridium, rhenium, rhodium, ruthenium, 0 to 5.0 % boron, cobalt, chromium, iron, germanium, niobium, nickel, silicon, tantalum, titanium, manganese, vanadium, the indicated percentages being percentages by weight.

Within the indicated limits, test series have shown that a suitable dental alloy having an increased stability is composed of 38.0 to 62.0 % silver, 4.5 to 11.0 % indium, 5 to 35.0 % gold, 0 to 20.0 % platinum, 8.0 to 35.0 % palladium, 0 to 7.0 % zinc, tin, gallium, copper, 0 to 2.0 % iridium, rhenium, rhodium, ruthenium, boron, cobalt, chromium, iron, germanium, niobium, nickel, silicon, tantalum, titanium, manganese, vanadium.

Further useful alloys are composed of 40.0 to 62.0 % silver, 5.0 to 10.0 % indium, 5 to 33.0 % gold, 0 to 15.0 % platinum, 10.0 to 32.0 % palladium, 0 to 7.0 % zinc, tin, gallium, copper, 0 to 2.0 % iridium, rhenium, rhodium, ruthenium, boron, cobalt, chromium, iron, germanium, niobium, nickel, silicon, tantalum, titanium, manganese, vanadium.

A further alloy may have the following composition: 45.0 to 62.0 % silver, 5.0 to 10.0 % indium, 5 to 20.0 % gold, 0 to 15.0 % platinum, 20.0 to 35.0 % palladium, 0 to 7.0 % zinc, tin, gallium, copper, 0 to 2.0 % iridium, rhenium, rhodium, ruthenium, boron, cobalt, chromium, iron, germanium, niobium, nickel, silicon, tantalum, titanium, manganese, vanadium.

A preferred alloy with gold content and having an esthetic yellow color is composed of 38.0 to 55.0 % silver, 5.0 to 10.0 % indium, 20.0 to 35.0 % gold, 0 to 15.0 % platinum, 8.0 to 20.0 % palladium, 0 to 7.0 % zinc, tin, gallium, copper, 0 to 2.0 % iridium, rhenium, rhodium, ruthenium, boron, cobalt, chromium, iron, germanium, niobium, nickel, silicon, tantalum, titanium, manganese, vanadium.

The gold content improves the corrosion resistance, hence counteracts the tendency of increased corrosion caused by the lower content of palladium as well as by the presence of indium and palladium in combination. On the other hand, gold, palladium and indium together yield a desirable increase in hardness.

Copper may also be present in an amount of at most 0.5 %, or be totally absent.

The composition and characteristic physical properties of exemplary alloys according to the invention are shown in the Tables 1 and 2 below.

**Table 1**

| Alloy | I | II | III | IV |
|---|---|---|---|---|
| Component | Fraction | Fraction | Fraction | Fraction |
| | % | % | % | % |
| Silver | 42 | 42 | 45 | 42 |
| Gold | 32 | 32 | 32 | 32 |
| Palladium | 14.98 | 15 | 14.8 | 14.8 |
| Indium | 9 | 9 | 5 | 9 |
| Platin | 2 | 2 | 2 | 2 |
| Ruthenium | 0.02 | | 0.2 | 0.2 |
| Gallium | | | 1 | |
| Boron | | | | |
| Tin | | | | |

**Table 2**

| Physical Property | Alloy I | Alloy II | Unit |
|---|---|---|---|
| Melting interval | 990-1070 | 990-1070 | °C |
| Density | 12.7 | 12.7 | g/cm³ |
| Elasticity module | 100 | 100 | GPa |
| Thermal expansion (25-500 °C) | 17*10⁻⁶ | 17*10⁻⁶ | K⁻¹ |
| Colour | pale yellow | pale yellow | |
| Hardness HV₅ after cast | 190 | 190 | |
| Elongation A5 | 6.9 | 4.5 | % |
| Corrosion: Release of ions (ISO 10271) | 1.34 | 3.6 | 10⁻⁶ g/cm² during 7 days |
| Melting interval | 995 - 1080 | 990-1075 | °C |
| Thermal expansion (25-500 °C) | 17.2*10⁻⁶ | 16.9*10⁻⁶ | K⁻¹ |
| Colour | pale yellow | pale yellow | |

In summary, it will be noted that the suggested alloys
a) correspond to the ISO 9693:1999 and ISO 8891:1998 standards, i.e. that they are both dental casting alloys and restorative metal-ceramic alloys suitable for veneering mainly but not exclusively with low-fusing ceramics having a high coefficient of thermal expansion,
b) offer very good mechanical properties, particularly a high stability, even after the firing of low-fusing, highly expanding ceramic substances;
c) a high firing stability in the firing cycles of the ceramic materials;
d) a very high corrosion and tarnishing resistance in spite of the high silver content;
e) are capable of fulfilling high esthetic requirements;
f) particularly in view of the incorporation of indium, surprisingly, it has been found that the alloys do not show the susceptibility to corrosion as experienced with low price .alloys of the silver palladium type with reduced gold and high indium content which had been marketed in the 1980's.

From the description and the exemplary embodiments, the one skilled in the art is enabled to derive variations of the invention without leaving the scope of protection which is defined by the claims.

## Claims

1. Ceramics-fused-to-metal dental alloy with silver content, composed of 35.5 to 62.5 % silver, 4.1 to 11.0 % indium, 5.0 to 37.0 % gold, 0 to 40.0 % platinum, 0.0 to 35.0 % palladium, totally 0 to 9.5 % zinc, tin, gallium, and/or copper, totally 0 to 2.0 % iridium, rhenium, rhodium, and/or ruthenium, totally 0 to 5.0 % boron, cobalt, chromium, iron, germanium, niobium, nickel, silicon, tantalum, titanium, manganese, and/or vanadium, the indicated percentages being percentages by weight.

2. The dental alloy of claim 1, composed of 38.0 to 62.0 % silver, 4.5 to 11.0 % indium, 5 to 35.0 % gold, 0 to 20.0 % platinum, 8.0 to 35.0 % palladium, totally 0 to 7.0 % zinc, tin, gallium, and/or copper, totally 0 to 2.0 % iridium, rhenium, rhodium, ruthenium, boron, cobalt, chromium, iron, germanium, niobium, nickel, silicon, tantalum, titanium, manganese, and/or vanadium, the indicated percentages being percentages by weight.

3. The dental alloy of claim 1, composed of 40.0 to 62.0 % silver, 5.0 to 10.0 % indium, 5 to 33.0 % gold, 0 to 15.0 % platinum, 10.0 to 32.0 % palladium, totally 0 to 7.0 % zinc, tin, gallium, and/or copper, totally 0 to 2.0 % iridium, rhenium, rhodium, ruthenium, boron, cobalt, chromium, iron, germanium, niobium, nickel, silicon, tantalum, titanium, manganese, and/or vanadium.

4. The dental alloy of claim 1, composed of 45.0 to 62.0 % silver, 5.0 to 10.0 % indium, 5 to 20.0 % gold, 0 to 15.0 % platinum, 20.0 to 35.0 % palladium, totally 0 to 7.0 % zinc, tin, gallium, and/or copper, totally 0 to 2.0 % iridium, rhenium, rhodium, ruthenium, boron, cobalt, chromium, iron, germanium, niobium, nickel, silicon, tantalum, titanium, manganese, and/or vanadium.

5. The alloy of one of claims 1 to 4, wherein the gold content is at least 10 %.

6. The alloy of claim 5, wherein the gold contents is at least 20 %.

7. The dental alloy of claim 1, composed of 38.0 to 55.0 % silver, 5.0 to 10.0 % indium, 20.0 to 35.0 % gold, 0 to 15.0 % platinum, 8.0 to 20.0 % palladium, totally 0 to 7.0 % zinc, tin, gallium, and/or copper, totally 0 to 2.0 % iridium, rhenium, rhodium, ruthenium, boron, cobalt, chromium, iron, germanium, niobium, nickel, silicon, tantalum, titanium, manganese and/or vanadium.

8. The alloy of one of claims 1 to 7, wherein manganese is absent.

9. The alloy of one of claims 1 to 8, wherein the content of copper is at most 0.5 % and preferably copper is absent.

10. Use of the alloy of one of claims 1 to 9 for producing dental prosthetic parts, wherein a layer of ceramic material is fused to a body essentially consisting of the alloy.

11. Use of the alloy according to claim 10, wherein the ceramic material has a thermal expansion coefficient of at least 14.5 * 10⁻⁶ K⁻¹, preferably within the range of (14.5 to 16.5) * 10⁻⁶ K⁻¹.

12. A dental prosthetic part obtained according to claim 10 or 11.

## Patentansprüche

1. Silberhaltige, aufbrennfähige Dentallegierung, bestehend aus 35,5 - 62,5 % Silber, 4,1 - 11,0 % Indium, 5,0 - 37,0 % Gold, 0 - 40,0 % Platin, 0,0 - 35,0 % Palladium, insgesamt 0 - 9,5 % Zink, Zinn, Gallium und/oder Kupfer, insgesamt 0 - 2,0 % Iridium, Rhenium, Rhodium und/oder Ruthenium, insgesamt 0 - 5,0 % Bor, Kobalt, Chrom, Eisen, Germanium, Niob, Nickel, Silizium, Tantal, Titan, Mangan und/oder Vanadium, wobei die Prozentangaben Gewichtsprozente sind.

2. Dentallegierung nach Anspruch 1, bestehend aus 38,0 - 62,0 % Silber, 4,5 - 11,0 % Indium, 5 - 35,0 % Gold, 0 - 20,0 % Platin, 8,0 - 35 % Palladium, insgesamt 0 - 7,0 % Zink, Zinn, Gallium und/oder Kupfer, insgesamt 0 - 2,0 % Iridium, Rhenium, Rhodium, Ruthenium, Bor, Kobalt, Chrom, Eisen, Germanium, Niob, Nickel, Silizium, Tantal, Titan, Mangan und/oder Vanadium, wobei die Prozentangaben Gewichtsprozente sind.

3. Dentallegierung nach Anspruch 1, bestehend aus 40,0 - 62,0 % Silber, 5,0 - 10,0 % Indium, 5 - 33,0 % Gold, 0 - 15,0 % Platin, 10,0 - 32,0 % Palladium, insgesamt 0 - 7,0 % Zink, Zinn, Gallium und/oder Kupfer, insgesamt 0 - 2,0 % Iridium, Rhenium, Rhodium, Ruthenium, Bor, Kobalt, Chrom, Eisen, Germanium, Niob, Nickel, Silizium, Tantal, Titan, Mangan und/oder Vanadium.

4. Dentallegierung nach Anspruch 1, bestehend aus 45,0 - 62,0 % Silber, 5,0 - 10,0 % Indium, 5 - 20,0 % Gold, 0 - 15,0 % Platin, 20,0 - 35,0 % Palladium, insgesamt 0 - 7,0 % Zink, Zinn, Gallium und/oder Kupfer, insgesamt 0 - 2,0 % Iridium, Rhenium, Rhodium, Ruthenium, Bor, Kobalt, Chrom, Eisen, Germanium, Niob, Nickel, Silizium, Tantal, Titan, Mangan und/oder Vanadium.

5. Legierung nach einem der Ansprüche 1 bis 4, in welcher der Goldgehalt mindestens 10 % beträgt.

6. Legierung nach Anspruch 5, in welcher der Goldgehalt mindestens 20 % beträgt.

7. Dentallegierung nach Anspruch 1, bestehend aus 38,0 - 55,0 % Silber, 5,0 - 10,0 % Indium, 20,0 - 35,0 % Gold, 0 - 15,0 % Platin, 8,0 - 20,0 % Palladium, insgesamt 0 - 7,0 % Zink, Zinn, Gallium und/oder Kupfer, insgesamt 0 - 2,0 % Iridium, Rhenium, Rhodium, Ruthenium, Bor, Kobalt, Chrom, Eisen, Germanium, Niob, Nickel, Silizium, Tantal, Titan, Mangan und/oder Vanadium.

8. Legierung nach einem der Ansprüche 1 bis 7 ohne Mangan.

9. Legierung nach einem der Ansprüche 1 bis 8, in welcher der Kupfergehalt höchstens 0,5 % beträgt und bevorzugt Kupfer abwesend ist.

10. Verwendung der Legierung nach einem der Ansprüche 1 bis 9 zur Herstellung von Zahnprothesenteilen, wobei eine Keramikschicht auf einen im wesentlichen aus der Legierung bestehenden Körper aufgebrannt wird.

11. Verwendung der Legierung nach Anspruch 10, wobei die Keramik einen Wärmeausdehnungskoeffizienten von mindestens 14,5 * 10⁻⁶ K⁻¹ aufweist, bevorzugt im Bereich von (14,5 bis 16,5) * 10⁻⁶ K⁻¹.

12. Gemäss Anspruch 10 oder 11 hergestelltes Zahnprothesenteil.

## Revendications

1. Alliage dentaire céramo-métallique à teneur en argent, composé de 35,5 à 62,5 % d'argent, de 4,1 à 11,0 % d'indium, de 5 à 37,0 % d'or, de 0 à 40,0 % de platine, de 0,0 à 35,0 % de palladium, d'un total de 0 à 9,5 % de zinc, étain, gallium, et/ou cuivre, d'un total de 0 à 2,0 % d'iridium, rhénium, rhodium, et/ou ruthénium, d'un total de 0 à 5,0 % de bore, cobalt, chrome, fer, germanium, niobium, nickel, silicium, tantale, titane, manganèse, et/ou vanadium, les pourcentages indiqués étant des pourcentages en poids.

2. Alliage dentaire selon la revendication 1, composé de 38,0 à 62,0 % d'argent, de 4,5 à 11,0 % d'indium, de 5 à 35,0 % d'or, de 0 à 20,0 % de platine, de 8.0 à 35.0 % de palladium, d'un total de 0 à 7,0 % de zinc, étain, gallium, et/ou cuivre, d'un total de 0 à 2,0 % d'iridium, rhénium, rhodium, ruthénium, bore, cobalt, chrome, fer, germanium, niobium, nickel, silicium, tantale, titane, manganèse, et/ou vanadium, les pourcentages indiqués étant des pourcentages en poids.

3. Alliage dentaire selon la revendication 1, composé de 40,0 à 62,0 % d'argent, de 5,0 à 10,0 % d'indium, de 5 à 33,0 % d'or, de 0 à 15,0 % de platine, de 10,0 à 32,0 % de palladium, d'un total de 0 à 7,0 % de zinc, étain, gallium, et/ou cuivre, d'un total de 0 à 2,0 % d'iridium, rhénium, rhodium, ruthénium, bore, cobalt, chrome, fer, germanium, niobium, nickel, silicium, tantale, titane, manganèse, et/ou vanadium.

4. Alliage dentaire selon la revendication 1, composé de 45,0 à 62,0 % d'argent, de 5,0 à 10,0 % d'indium, de 5 à 20,0 % d'or, de 0 à 15,0 % de platine, de 20,0 à 35,0 % de palladium, d'un total de 0 à 7,0 % de zinc, étain, gallium, et/ou cuivre, d'un total de 0 à 2,0 % d'iridium, rhénium, rhodium, ruthénium, bore, cobalt, chrome, fer, germanium, niobium, nickel, silicium, tantale, titane, manganèse, et/ou vanadium.

5. Alliage selon l'une des revendications 1 à 4, où la teneur en or est égale à au moins 10 %.

6. Alliage selon la revendication 5, où la teneur en or est égale à au moins 20 %.

7. Alliage dentaire selon la revendication 1, composé de 38,0 à 55,0 % d'argent, de 5,0 à 10,0 % d'indium, de 20,0 à 35,0 % d'or, de 0 à 15,0 % de platine, de 8,0 à 20,0 % de palladium, de 0 à 7,0 % de zinc, étain, gallium, cuivre, de 0 à 2,0 % d'iridium, rhénium, rhodium, ruthénium, bore, cobalt, chrome, fer, germanium, niobium, nickel, silicium, tantale, titane, manganèse, et/ou vanadium.

8. Alliage selon l'une des revendications 1 à 7, où le manganèse est absent.

9. Alliage selon l'une des revendications 1 à 8, où la teneur en cuivre est égale à 0,5 % au plus et préférablement le cuivre est absent.

10. Utilisation de l'alliage selon l'une des revendications 1 à 9 pour produire des éléments prothétiques dentaires, où une couche d'un matériau céramique est cuite sur un corps constitué essentiellement de l'alliage.

11. Utilisation de l'alliage selon la revendication 9, où le matériau céramique présente un coefficient d'expansion thermique d'au moins 14,5 * 10⁻⁶ K⁻¹, préférablement dans la plage de (14,5 à 16,5) * 10⁻⁶ K⁻¹.

12. Elément prothétique dentaire obtenu selon la revendication 10 ou 11.
